# EUROPÄISCHE PATENTANMELDUNG

(11) **EP 3 576 098 A1**
(43) Veröffentlichungstag der Anmeldung: **04.12.2019**
(21) Anmeldenummer: 18175203.1
(22) Anmeldetag: 30.05.2018
(51) Int. Cl.: G16H 40/60, A61F 2/00, G16H 20/30

(54) **TRAININGSVORRICHTUNG ZUM TRAINIERENDEN STIMULIEREN VON NERVENZELLENENDEN UND EINE ENTSPRECHENDE PROTHESE**

(71) Anmelder: Saphenus Medical Technology GmbH, 3500 Krems an der Donau (AT)
(72) Erfinder: LANG, Bernhard, 1220 Wien (AT); BRANDSTÄTTER, Martin, 1090 Wien (AT); PITSCHL, Aaron, 1040 Wien (AT); SCHULTHEIS, Rainer, 1040 Wien (AT)
(74) Vertreter: Patentbüro Paul Rosenich AG

(57) **Zusammenfassung**

Der Gegenstand der Erfindung betrifft eine Trainingsvorrichtung 10 zum trainierenden Stimulieren von Nervenzellenenden umfassend, ein Gehäuse 11, wobei das Gehäuse 11 zumindest einen ersten Gehäuseabschnitt 12 aufweist sowie mehrere Stimulatoren 20 zum Stimulieren von physionomietypischen Nervenarealabschnitten. Zumindest am ersten Gehäuseabschnitt 12 des Gehäuses 11 sind mehrere Stimulatoren 20 systematisch angeordnet, sodass die mehrere Stimulatoren 20 im Trainingszustand auf deren jeweils zugeordneten Nervenzellenenden einwirken. Weiters betrifft der Gegenstand der Erfindung ein Verfahren zum Betreiben der Trainingsvorrichtung 10, die Verwendung der Trainingsvorrichtung 10 als modulare Stimulationsvorrichtung zum Stimulieren von Nervenzellenenden sowie eine Prothese, ein Verfahren zum Herstellen einer Prothese und die Verwendung einer Trainingsvorrichtung 10 zum Stimulieren von Nervenzellenenden in einer Prothese.

## Beschreibung

Die Erfindung betrifft eine Trainingsvorrichtung zum trainierenden Stimulieren von Nervenzellenenden, eine Prothese sowie ein Verfahren zum Betreiben einer Trainingsvorrichtung und ein Verfahren zum Herstellen eine Prothese sowie die Verwendung einer Trainingsvorrichtung zum Stimulieren von Nervenzellenenden und die Verwendung einer Trainingsvorrichtung in einer Prothese nach den Oberbegriffen der unabhängigen Ansprüche.

Am 8.Juni 2015 wurde durch die Tageszeitung derStandard.at unter Wissenschaft > Mensch ein Artikel publiziert, der Auskunft über eine Entwicklung von Professor Hubert Egger, einem der weltweit führenden Prothesenforschern gibt. Es wird dort über die erste Beinprothese berichtet, die «mitfühlt». Ausführlich wird dort dargelegt, dass bei einem beinamputierten Patienten abgetrennte Nerven reaktiviert wurden und in ein Hautareal am Beinstumpf verlegt wurden. Da dieses Hautareal anschliessend die reinnervierten Nervenzellenenden aufweist, wird diese Hautareal besonders empfindlich. Die Nervenenden wurden dabei so verlegt, dass in diesem Hautareal durch die dorthin verlegten Nervenenden der ehemalige Fuss mit seinen physionomietypischen Nervenarealabschnitten abgebildet wurde. Ein physionomietypischer Nervenarealabschnitt ist mit anderen Worten ein natürlicher Nervenarealabschnitt, welcher an die Oberfläche eines dafür eigentlich untypischen Hautareals verlegt wird. Der Patient fühlte daher an diesem Hautareal nicht (nur) wie üblich die dort befindliche Haut(oberfläche), sondern die reinnervierten Nervenzellenenden und somit quasi seine Fusssohle, die ja infolge Amputation der Extremität gar nicht mehr vorhanden ist.

Die bei der vorgestellten Lösung verwendete Prothese waren so ausgebildet, dass die Stimulatoren im Schaft der Prothese fixiert angeordnet sind, sodass erst nach dem individuellen, orthoptischen Anpassen des Prothesenschafts am Patienten eine Stimulation der Nervenzellenenden möglich ist.

Es ist die Aufgabe der vorliegenden Erfindung einen oder mehrere Nachteile des Standes der Technik zu beheben. Ein bekanntes Phänomen ist, dass reinnervierte Nervenzellenenden - da sie ja an einem in einem für sie an sich fremden Gebiet zu liegen kommen, relativ lange brauchen, bis sie voll ansprechbar sind. Insbesondere soll daher eine Trainingsvorrichtung zum trainierenden Stimulieren von Nervenzellenenden geschaffen werden, mit welcher es postoperativ zeitnah möglich ist, eine natürliche Bewegung einer fehlenden Extremität an den Nervenzellenenden des Patienten gefühlsecht zu trainieren. Damit soll geholfen werden, möglichst rasch insbesondere durch Nerven-Zell-Wachstum (Zellteilung an den Synapsen) die volle Funktionalität der betroffenen Nervenzellenenden herzustellen. Weiter soll ein Verfahren zum Betreiben einer Trainingsvorrichtung sowie eine Prothese für den Einsatz dieser Trainingsvorrichtung, ein Verfahren zum Herstellen dieser Prothese und die Verwendung der Trainingsvorrichtung zum trainierenden Stimulieren sowie die Verwendung der Trainingsvorrichtung in einer Prothese für einen anderen Zweck bereitgestellt werden. Dadurch sollen, welche ein oder mehrere Nachteile des Standes der Technik behoben werden und die für den angestrebten Zweck nämlich die Wachstumsanregung bzw. Verbesserung der Sensibilität der Nervenzellenenden besser gesorgt werden können.

Die Aufgabe wird durch die Merkmale der unabhängigen Ansprüche gelöst. Vorteilhafte Weiterbildungen sind in den Figuren, der zugehörigen Beschreibung und in den abhängigen Patentansprüchen dargelegt.

Die erfindungsgemässe Trainingsvorrichtung zum trainierenden Stimulieren von Nervenzellenenden umfasst, ein Gehäuse mit zumindest einem ersten Gehäuseabschnitt aufweist sowie mit mehreren Stimulatoren zum Stimulieren von physionomietypischen Nervenarealabschnitten. Dabei sind zumindest am ersten Gehäuseabschnitt des Gehäuses mehrere Stimulatoren systematisch angeordnet, sodass die mehrere Stimulatoren im Trainingszustand auf die ihnen jeweils zugeordneten Nervenzellenenden einwirken können.

Die Systematik der Anordnung hängt dabei von der tatsächlichen Lage der jeweiligen relevanten Nervenzellenenden am postoperativ bereitgestellten physionomietypischen Nervenarealabschnitt ab, so dass ein sequenzielles Ansprechen des jeweiligen Stimulators der mehreren Stimulatoren beim Patienten das Gefühl einer echten Stimulation entlang des tatsächlichen bzw. natürlichen Hautarealabschnitts der fehlenden Extremität erzeugt und im Gehirn als solches wahrgenommen wird. Beim Einwirken der mehreren Stimulatoren auf die ihnen zugeordneten Nervenzellenenden werden Stimulationssignale von den Stimulatoren auf die Nervenzellenenden übertragen. Damit werden die Nervenzellenenden frühestmöglich trainiert, sodass die Eingewöhnungsphase des Patienten an eine später folgende Prothese zeitlich minimiert wird.

Als Stimulieren wird hier immer auch eine Übertragung von Informationen bzw. Informationsübertragung verstanden, wobei typischerweise keine Energieübertragung von der Trainingsvorrichtung an den Patienten erfolgt.

Mit der erfindungsgemässen Trainingsvorrichtung kann unmittelbar nach einer Operation somit beispielsweise beim Patienten das Gefühl des Gehens mit seinem (ehemaligen) Fuss trainingshalber simuliert werden. Durch diese Trainingsvorrichtung ist es möglich postoperativ das Wachstum der physionomietypischen Nervenarealabschnitte bzw. Nervenzellenenden zu stimulieren und somit zu einer zeitnahen und optimalen Bereitstellung eines geeigneten Hautareals am verbleibenden Teil der Extremität des Patienten zu kommen. In anderen Worten dient die Trainingsvorrichtung somit der erfolgreicheren Reinnervation von Nervenzellenenden für den Zweck der späteren Anpassung eines am Patienten verbliebenen Teil einer Extremität an eine Prothese.

Insbesondere sind mit der Trainingsvorrichtung reinnervierte Nervenzellenenden stimulierbar, wobei die reinnervierten Nervenzellenenden eines Fusses besonders gut trainierbar sind.

Bevorzugt bildet die systematische Anordnung der mehreren Stimulatoren ein Abbild der Ganglinie einer menschlichen Fusssohle ab. Dabei sind die mehreren Stimulatoren nach einer Matrix angeordnet, beispielsweise blitzförmig angeordnet. Beispielsweise werden dafür zumindest fünf Stimulatoren im ersten Gehäuseabschnitt benötigt, wodurch das Abbild der Ganglinie des menschlichen Fusses besonders gut abgebildet werden kann. Die systematische Anordnung der mehreren Stimulatoren erfolgt dabei so, dass jeder Stimulator jeweils zumindest an ein ihm zugeordnetes physionomietypische Nervenzellenende angeordnet wird, welches, bei einem intakten Fuss, mit den jeweiligen Nervenzellenende entlang der natürlichen Ganglinie des Fusses verbunden ist bzw. wäre.

Bevorzugt werden im Bereich jedes physionomietypischen Nervenzellenendes mehrere Stimulatoren, insbesondere zwei Stimulatoren angeordnet, wodurch die Stimulationsauflösung verbessert wird.

Insbesondere können gemäss einer besonderen Ausführung der Erfindung diese beiden Stimulatoren unterschiedlich ausgestaltet werden, so dass an der jeweiligen Stelle unterschiedliche Stimulationen bzw. Stimulationssignale an die Extremität bzw. an den Fuss übergeben werden. Beispielsweise kann das Signal des ersten Stimulators die Tatsache der Berührung signalisieren, während das Signal des Weiteren Stimulators erst ab einer bestimmten simulierten Druckintensität der Berührung zugeschaltet wird und so sowohl qualitative wie auch quantitative Informationen abgegeben werden können.

Die natürliche Ganglinie eines Fusses wird üblicherweise bei einer Ganglinienanalyse an einem dafür geeigneten Messplatz bestimmt. Dabei werden die Kinematik und die Kinetik des Ganges gemessen und entlang einer Linie an der Fusssohle dargestellt. Typischerweise verläuft eine natürliche Ganglinie weitgehend entlang der Längsstreckung der Fusssohle. Dabei kann, je nach individueller Anatomie des Fusses, jeder Fuss mehrere einzelne natürliche Ganglinien aufweisen, welche oftmals als eine einzige natürliche Ganglinie definiert werden. Beispielsweise unterscheiden sich die natürlichen Ganglinien eines gesunden Fusses von den Ganglinien eines Kunstfusses am gleichen Patienten hinsichtlich deren Ganglinienbreite, oder die einzige Ganglinie eines Trägers einer Oberschenkelprothese von der einzigen Ganglinie eines Trägers eine Unterschenkelprothese. Somit lässt sich mit der hier beschriebenen Trainingsvorrichtung auf den Patienten abgestimmte Ganglinien bzw. einzige Ganglinie individuell trainieren.

Dabei kommt es darauf an, wo die Kunstfusssohle die Ganglinie bei widmungsgemässer Verwendung aufweist. Die mehreren Stimulatoren an der Trainingsvorrichtung werden zu einem Abbild der Ganglinie, in dem sie an den Stellen angeordnet werden, die den entsprechenden Nervenarealendabschnitten entsprechen. Die reinnervierten Nervenzellenenden am physionomietypischen Nervenarealabschnitt können ebenfalls nach einem Abbild der Ganglinie angeordnet sein, wobei das aber nicht zwingend notwendig ist. Entscheidend ist lediglich die richtige Zuordnung von Stimulatoren und Nervenzellenenden.

Vorzugsweise ist eine Steuereinrichtung zum Steuern der mehreren Stimulatoren vorhanden, welche zumindest ein Trainingsprogramm umfasst. Damit werden die mehreren Stimulatoren, welche mit der Steuereinrichtung verbunden sind, nach einer vordefinierten Programmsequenz angeregt, sodass die Nervenzellenenden einzeln und auch im Kollektiv gezielt trainierbar sind, bzw. reproduzierbar trainierbar sind.

Bevorzugt ist die Steuereinrichtung mit einer Visualisierungseinrichtung gekoppelt, um dem Patienten eine Trainingsstimulation aus dem zumindest einen Trainingsprogramm optisch darzulegen. Damit werden während dem Training der Nervenzellenenden nicht nur die taktile Sensorik im Bereich des physionomietypischen Nervenarealabschnitt angeregt, sondern auch die visuellen Rezeptoren im menschlichen Auge angeregt, wodurch die gesamte Wahrnehmung und Verarbeitung im menschlichen Gehirn verbessert wird. Das Training kann gemäss einer der Überlegungen, die der Erfindung zugrunde liegen dadurch unterstützt werden, dass dem Gehirn über andere Sinneskanäle die gleiche Information zugeführt wird. Beispielsweise durch bildliche Darstellung an welcher Stelle (des "abgebildeten" Fusses) der jeweilige Trainingsreiz abgegeben wird.

Alternativ oder ergänzend können Nachrichten, wie beispielsweise Anweisungen von einem Dritten, beispielsweise einem Arzt, für den Patienten an der Visualisierungseinrichtung dargestellt werden.

Als einzelnes Trainingsprogramm wird hier vorwiegend ein Trainingsprogramm verstanden, welches Trainingsdaten bzw. Trainingsbefehle für bestimmte Bewegungsabläufe, wie beispielsweise Trainingsdaten zu Laufbewegungen, Hüpfbewegungen oder Bergsteigbewegungen umfasst. Alternativ kann beispielsweise - im Falle der Reinnervation von Fingernerven für eine menschliche Hand - eine Greifbewegung als Trainingsprogramm mit der Steuereinrichtung ausführbar sein.

Bevorzugt ist die Steuereinrichtung im Gehäuse angeordnet, wodurch ein kompakter Aufbau der Trainingsvorrichtung ermöglicht wird.

Bevorzugt ist die Steuereinrichtung mit einer externen Remoteeinrichtung, wie beispielsweise einer Fernbedienung oder ein Computer, steuerbar. Die Remoteeinrichtung kann mithilfe eines Datenkabels mit der Schnittstelle der Trainingsvorrichtung verbunden werden. Damit kann die Trainingsvorrichtung, welches sich an einem nur schwer zugänglichen Bereich des Körpers befindet, vom Anwender gesteuert werden.

Insbesondere ist die Steuereinrichtung mit einem mobilen Endgerät, wie beispielsweise einem Smartphone oder einem Tablet steuerbar. Dafür weist das mobile Endgerät eine Applikation auf, welches mit der Trainingsvorrichtung zum Austausch von Daten verbindbar ist. Damit ist kein zusätzliches Gerät notwendig und das Steuern der Trainingsvorrichtung mit einem für den Anwender bekanntes bzw. vertrautes Endgerät möglich.

Bevorzugterweise weist die Trainingsvorrichtung eine Datenübertragungseinrichtung zum Übertragen von Daten auf. Beispielsweise kann der Anwender Nachrichten, als Daten, an die Trainingsvorrichtung übertragen, welche an der Visualisierungseinrichtung angezeigt werden können.

Bevorzugt ist die Datenübertragungseinrichtung in der Steuereinrichtung angeordnet sodass externe Trainingsdaten auf die Steuereinrichtung übertragen werden können. Damit kann jeder berechtigte Anwender bestehende Trainingsdaten an der Trainingsvorrichtung adaptieren oder neue Trainingsprogramme auf die Trainingsvorrichtung übertragen.

Als Anwender wird hier sowohl ein Patient, als auch ein Physiotherapeut, und/oder ein Orthopäde verstanden.

Alternativ oder ergänzende werden mit der Datenübertragungseinrichtung gegebenenfalls Stimulationsdaten auf die Steuereinrichtung übertragen. Als Stimulationsdaten bzw. Stimulationsbefehle werden jene Daten verstanden, welche einzelne reinnervierte Nervenzellenenden individuell stimulieren können. Damit werden unterschiedlich empfindliche Nervenzellenenden im physionomietypischen Nervenarealabschnitt zu einem gleich hohen Sensibilitätsniveau trainierbar.

Bevorzugt weist die Datenübertragungseinrichtung eine Sendeeinheit und eine Empfangseinheit zum drahtlosen Datenaustausch auf. Typischerweise umfasst die Datenübertragungseinrichtung dafür eine WLAN-Einheit oder eine Bluetooth® - Verbindung, womit die Trainingsdaten und Stimulationsdaten einfach auf die Steuereinrichtung übertragen werden können.

Vorzugsweise umfasst die Datenübertragungseinrichtung eine Schnittstelle, auf dem ein Anwender Trainingsprogramme definieren kann. Dabei kann typischerweise eine Eingabeeinrichtung, wie beispielsweise eine Tastatur, mit der Schnittstelle verbunden werden, sodass die Trainingsprogramme einfach adaptierbar sind.

Alternativ oder ergänzend kann ein Anwender an der Schnittstelle Trainingsprogramme und Stimulationsprogramme definieren. Stimulationsprogramm umfassen Stimulationsbefehle bzw. Stimulationsdaten zum Stimulieren einzelner Nervenzellenenden, welche somit einzeln adaptierbar sind. Beispielsweise können somit Trainingsprogramme und Stimulationsprogramme synchron adaptiert werden.

Insbesondere umfasst die Schnittstelle eine Eingabeeinrichtung zum Eingeben von Stimulationsdaten, Trainingsdaten oder Sensordaten. Als Eingabeeinrichtung ist neben einem Hauptschalter (Ein- und Ausschalter) ein Auswahlschalter zum Auswählen von vorgegebenen Trainingsprogrammen und/oder Stimulationsprogrammen vorgesehen. Damit wird die Aktivierung der Trainingsvorrichtung bzw. die Auswahl der Trainingsprogramme und/oder der Stimulationsprogramme für den Anwender vereinfacht.

Bevorzugt ist die Eingabeeinrichtung an der Visualisierungseinrichtung angeordnet, wodurch die Haptik der Trainingsvorrichtung für den Anwender verbessert wird.

Insbesondere ist die Eingabeeinrichtung in der Visualisierungseinrichtung als Touchscreen integriert. Damit lässt sich eine besonders leichte Trainingsvorrichtung realisieren.

Bevorzugterweise ist die Datenübertragungseinrichtung mit externen Sensordaten einer Prothese versorgbar. Dabei werden externe Sensordaten an die Datenübertragungseinrichtungen übertragen und in der damit verbunden Steuereinrichtung zum Steuern der mehreren Stimulatoren verwendbar. Beispielsweise können damit externen Sensordaten, wie beispielsweise Drucksensordaten, von einem Prothesenteil, wie bevorzugt von einer Kunstfusssohle, an die Datenübertragungseinrichtung gesendet werden und an die mehreren Stimulatoren übertragen werden.

Alternativ oder ergänzend ist die Datenübertragungseinrichtung mit mehreren externen Sensoren verbunden. Damit lassen sich neben den Sensordaten von einer Prothese auch weitere Daten von externen Sensoren, wie beispielsweise einem Temperatursensor, Luftfeuchtesensor, einem GPS-Sensor usw. versorgen, welche in Kombination mit den Sensordaten der Prothese zum trainieren der Nervenzellenenden herangezogen werden können. Beispielsweise können GPS-Daten verwendet werden, um den Standort des Prothesenträgers festzuhalten und mit einem Trainingsprogramm in der Trainingsvorrichtung zu kombinieren. Damit kann das Trainingsprogramm "Bergsteigen", oder "Laufen", aber auch "Tanzen" mit dem aktuellen oder mit angestrebten Standort(en) des Prothesenträgers kombiniert werden.

Bevorzugt ist die Datenübertragungseinrichtung mit einer Cloud in einem Netzwerk zum Austauschen von Daten verbunden, wobei die Daten externe Sensordaten, externe Trainingsdaten oder externe Stimulationsdaten sein können. Dies ermöglicht den Anwender Zugriff zu historischen Daten, welche sich in der Cloud befinden. Mit der Verwendung der historischen Daten können beispielsweise Wartungsarbeiten bzw. Serviceapplikationen in der Trainingsvorrichtung zeitlich verkürzt bzw. verbessert werden.

Bevorzugt ist die Datenübertragungseinrichtung mit einem Online-Dienst zum Austauschen von Daten verbunden, wodurch die Zugriffsberechtigungen der Anwender einfach vergeben werden können.

Bevorzugt werden die Daten mithilfe eine Blockchain - Technologie abgespeichert, wodurch die Daten besonders gut verschlüsselt gespeichert sind, sodass keine sensiblen bzw. persönlichen Daten des Anwenders veröffentlicht werden können.

Vorzugsweise weist die Steuereinrichtung eine Recheneinheit auf, wobei die Recheneinheit zumindest ein Stimulationsprogramm zum Erstellen von Stimulationsbefehlen für einen Stimulationszustand aufweist. Damit können individuelle Stimulationsbefehle in der Steuereinrichtung erstellt werden.

Alternativ oder ergänzend weist die Recheneinheit zumindest ein Trainingsprogramm zum Erstellen von Trainingsbefehlen für einen Trainingsvorgang auf. Damit können neben individuellen Stimulationsbefehle auch individuelle Trainingsbefehle in der Steuereinrichtung erstellt werden und miteinander kombiniert werden.

Bevorzugt ist die Recheneinheit mit der Datenübertragungseinrichtung zum Austausch von Daten verbunden, wodurch das Erstellen neuer Stimulationsbefehle oder Trainingsbefehle möglich ist.

Insbesondere ist die Recheneinheit mit der Datenübertragungseinrichtung zum Austausch von Sensordaten externer Sensoren verbunden. Dadurch lassen sich beispielsweise Sensordaten von an einer Prothese angeordnete Sensoren und GPS-Daten mit den Trainingsdaten kombinieren.

Insbesondere weist die Recheneinheit einen Rechenalgorithmus auf, sodass die Stimulationsbefehle und/oder die Trainingsbefehle reproduzierbar erzeugt werden können.

Insbesondere ist der Rechenalgorithmus ein selbstlernender Rechenalgorithmus, wodurch historische Daten und aktuelle Daten in der Trainingsvorrichtung einfach miteinander verknüpft werden können und die Trainingsprogramme bzw. die Stimulationsprogramme sich somit selbständig verbessern.

Bevorzugt verarbeitet der Rechenalgorithmus im Betriebszustand externe Sensordaten von externen Sensoren einer Kunstfusssohle, wodurch der Patient vorab, also ohne eine Kunstfusssohle zu besitzen, mit einer für seine Umstände passende Kunstfusssohle trainiert werden kann.

Bevorzugt weist die Steuereinrichtung eine Speichereinheit auf. Damit lassen sich Trainingsprogramme, Stimulationsprogramme, Trainingsdaten, Stimulationsdaten, Trainingsbefehle bzw. Stimulationsbefehle in der Trainingsvorrichtung speichern.

Bevorzugterweise weist das Gehäuse der Trainingsvorrichtung zumindest einen zweiten Gehäuseabschnitt auf, wobei am zweiten Gehäuseabschnitt eine Montageeinheit zum Montieren des Gehäuses an einem ersten Prothesenteil einer Prothese vorgesehen ist. Mit einem Schienensystem, einem Klipssystem, oder Bajonettverschlusssystem als Montageeinheit lässt sich die Trainingsvorrichtung reproduzierbar an einer Prothese, insbesondere an einem Prothesenschaft, anordnen.

Bevorzugt ist an der Trainingsvorrichtung eine Positioniereinheit zum Positionieren der Trainingsvorrichtung an dem ersten Prothesenteil einer Prothese vorgesehen. Damit lassen sich die mehreren Stimulatoren der Trainingsvorrichtung genau an deren zugeordneten reinnervierten Nervenzellenenden am physionomietypischen Nervenarealabschnitt positionieren. Beispielsweise wird ein Anschlag oder eine Positionierschraube als Positioniereinheit verwendet.

Bevorzugt umfasst das Gehäuse zumindest einen weiteren Gehäuseabschnitt mit der Visualisierungseinrichtung, wodurch der Anwender der Trainingsvorrichtung zumindest einzelne Trainingsdaten bzw. Trainingsbefehle sowie Stimulationsdaten bzw. Stimulationsbefehle und Informationen zur Trainingsvorrichtung an der Visulisierungseinrichtung ablesen kann. Typischerweise umfassen diese Informationen zur Trainingsvorrichtung Informationen zum Betriebszustand der Trainingsvorrichtung und beinhalten gegebenenfalls einzelne Aufforderungen für den Anwender der Trainingsvorrichtung.

Bevorzugt lässt sich auf der Visualisierungseinrichtung eine Darstellung des physionomietypischen Nervenarealabschnitts samt reinnervierten Nervenzellenenden darstellen, wodurch neben der taktilen Wahrnehmung auch visuelle Rezeptoren im Gehirn des Anwenders trainiert werden.

Alternativ oder ergänzend lässt sich auf einem mobilen Endgerät eine Darstellung des physionomietypischen Nervenarealabschnitts samt reinnervierten Nervenzellenenden darstellen. Damit benötigt die Trainingsvorrichtung keine Visualisierungseinrichtung, womit die Trainingsvorrichtung einen kompakteren Aufbau aufweist und somit die Haptik der Trainingsvorrichtung verbessert wird.

Alternativ oder ergänzend lässt sich eine animierte Darstellung des physionomietypischen Nervenarealabschnitts samt reinnervierten Nervenzellenenden gemeinsam mit der systematischen Anordnung der mehreren Stimulatoren darstellen. Damit kann der Anwender Stimulationen einzelner Stimulatoren an den jeweiligen reinnervierten Nervenzellenenden in animierter Weise beobachten und seinen empfundenen Gefühlen/Empfindungen zuordnen, wodurch die taktilen Rezeptoren und die visuellen Rezeptoren des Anwenders verbessert trainiert werden und im Gehirn des Anwenders verknüpft werden.

Alternativ oder ergänzend umfasst der eine weiter Gehäuseabschnitt eine Energiespeichereinheit. Damit lässt sich ein kompakter Aufbau im Gehäuse der Trainingseinrichtung realisieren und eine Batterie oder ein Akkumulator als Energiespeichereinheit so anordnen, dass eine gleichmässige Gewichtsverteilung im Gehäuse möglich wird. Dadurch wird der Trainingskomfort für den Patienten verbessert.

Vorzugsweise ist die Steuereinrichtung so ausgerüstet, dass wahlweise ein Trainingsprogramm für Trainingszwecke oder ein Stimulationsprogramm zur Stimulation mittels externer Sensoren einstellbar ist. Damit ist die Trainingsvorrichtung für diverser Zwecke multifunktional verwendbar.

Insbesondere erkennt die Steuereinrichtung automatisch einen Rehabilitationsmodus (reha mode) und einen Prothesenmodus (aktive mode) mittels der Erkennungseinrichtung, welche an der Trainingsvorrichtung angeordnet ist. Damit wird dem Anwender die Entscheidung des aktuell notwendigen Programms in der Trainingsvorrichtung abgenommen.

Bevorzugt ist die Erkennungseinrichtung eine RFID - Einheit, wodurch die Trainingsvorrichtung den Verwendungszweck selbstständig erkennt, ohne dass beispielsweise eine Steckverbindung an der Trainingsvorrichtung angeschlossen werden muss.

Ein weiterer Aspekt der Erfindung betrifft ein Verfahren zum Betreiben einer hier vorliegend beschriebenen Trainingsvorrichtung, insbesondere als modulare Stimulationsvorrichtung, umfassend folgende Schritte:
- Anordnen der Trainingsvorrichtung an einem physionomietypischen Nervenarealabschnitt mit Nervenzellenenden, insbesondere mit reinnervierten Nervenzellenenden;
- Darstellen der Nervenzellenenden an einer Visualisierungseinrichtung der Trainingsvorrichtung oder an einem mobilen Endgerät.

Mit dem Verfahren zum Betreiben der hier beschriebenen Trainingsvorrichtung ist es möglich, postoperativ das Wachstum der relevanten Nervenarealabschnitte bzw. reinnervierten Nervenzellenenden bzw. deren Synapsen zu stimulieren. Dabei kann der Anwender den Trainingsfortschritt bzw. Stimulationsvorgang an der Visualisierungseinrichtung oder am mobilen Endgerät beobachten. Damit werden nicht nur die taktile Sensorik im menschlichen Körper des Anwenders angeregt, sondern auch die visuellen Rezeptoren, wodurch die gesamte Wahrnehmung und Verarbeitung im menschlichen Gehirn - und damit der Trainingserfolg - verbessert wird.

Bevorzugt wird zusätzlich zumindest der Trainingszustand bzw. der Aktivitätszustand wenigstens eines der mehreren Stimulatoren an der Visualisierungseinrichtung oder am mobilen Endgerät dargestellt, sodass der Anwender der Trainingseinrichtung das Auslösen einer bestimmten Stimulation mit einem bestimmten Stimulator geistig verknüpfen kann und damit der Trainingseffekt verbessert wird.

Bevorzugt erfolgt anschliessend ein Anpassen von zumindest einem Stimulationsbefehl, wodurch der Anwender die Möglichkeit hat, auf einzelne taktile Reizungen zu reagieren.

Alternativ oder ergänzend erfolgt anschliessend ein Anpassen von zumindest einem Trainingsbefehlen, wodurch der Anwender die Möglichkeit hat, auf einzelne taktile Reizungen zu reagieren und das Training von einzelne Nervenzellenenden selbstständig anzupassen.

Bevorzugt wird beim Anpassen von zumindest einem der Stimulationsbefehle oder von zumindest einem der Trainingsbefehle eines der mehreren Stimulationsprogramme oder eines der mehreren Trainingsprogramme ausgewählt. Der Anwender kann beispielsweise somit die Intensität der Stimulationen selbständig steuern oder das Trainingsprogramm nach seinen individuellen Bedürfnissen anpassen.

Ein weiterer Aspekt der Erfindung betrifft die Verwendung der hier beschriebenen Trainingsvorrichtung als modulare Stimulationsvorrichtung zum Stimulieren von Nervenzellenenden, insbesondere von reinnervierten Nervenzellenenden. Damit lässt sich das Wachstum bzw. die Sensibilität der Nervenzellenenden am verbleibenden Teil einer Extremität in einem sehr frühen Stadium nach dem Verlust der Extremität verbessern, sodass die Gewöhnungsphase an eine später notwendige Prothese mit Stimulationseinsatz verkürzt werden kann.

Bevorzugt erfolgt die Verwendung der hier vorliegend beschriebenen Trainingsvorrichtung mit dem hier vorliegend beschriebenen Verfahren zum Betreiben der Trainingsvorrichtung, wodurch auch die visuellen Rezeptoren im Gehirn des Anwenders angeregt werden und dadurch die Gewöhnungsphase weiter verkürzt wird.

Ein weiterer Aspekt der Erfindung betrifft eine Prothese mit einem ersten Prothesenteil, wobei der erste Prothesenteil zumindest eine Aufnahme zum Aufnehmen einer Trainingsvorrichtung als modulare Stimulationsvorrichtung aufweist und wobei die Trainingsvorrichtung separierbar/entfernbar an der zumindest einen Aufnahme des ersten Prothesenteils angeordnet ist.

Mit der erfindungsgemässen Prothese kann der Anwender einer Trainingsvorrichtung, welche unmittelbar nach einer Operation zum Verbessern des Wachstums bzw. der Sensibilität von relevanten Nervenarealabschnitte bzw.

Nervenzellenenden verwendet wurde, diese anschliessend zusammen mit der Prothese für den Trainingseinsatz, aber auch für den Sensoreinsatz verwenden.

Die Aufnahme ist erfindungsgemäss fensterartig ausgebildet und umfasst typischerweise einen Rahmen oder einen Einsatz, wobei die Trainingsvorrichtung darin eingesetzt wird. Beispielsweise kann somit der Anwender, welchem ein Fuss amputiert wurde die Trainingsvorrichtung nicht nur als Rehabilitationsmodul verwenden, sondern auch als eine modulare Stimulationsvorrichtung verwenden. Dabei ist die modulare Stimulationsvorrichtung mit einer Kunstfusssohle verbindbar, sodass beispieslweise eine Abrollbewegung eines Kunstfusses entlang seiner Ganglinie an die modulare Stimulationsvorrichtung rückgemeldet wird und dem Patienten somit das Gefühl des Gehens vermittelt werden kann (Sensoreinsatz). Damit wird nicht nur die Lebensqualität des Anwenders verbessert, sondern auch die bekanntlich hohen Kosten für Prothesenanpassungen und Rehabilitation gesamthaft minimiert.

Insbesondere ist der erste Prothesenteil ein Prothesenschaft, ein Prothesenstrumpf oder eine Prothesenmanschette, oder eine Prothesenkosmetik (Teil der Prothese, welcher sich zwischen dem Prothesenschaft und dem Prothesenfuss befindet). Je nach amputierter Extremität, wie beispielsweise Oberschenkel, Unterschenkel, Oberarm, oder Unterarm haben die genannten ersten Prothesenteile in der Anwendung Vorteile. Beispielsweise ist die Anordnung der modularen Stimulationsvorrichtung bzw. Trainingsvorrichtung individuell an den Anwender der Prothese anpassbar, sodass je nach Bedürfnis des Anwenders bzw. Tragekomfort der Prothesenvorrichtung die Aufnahme zum Aufnehmen der modularen Stimulationsvorrichtung an unterschiedlichen Stellen an der Prothese positionierbar.

Insbesondere ist die modulare Stimulationsvorrichtung die hier beschriebene Trainingsvorrichtu ng.

Vorzugsweise weist der erste Prothesenteil zumindest eine Eingabeeinheit zum Erkennen der Trainingsvorrichtung auf. Damit erkennt die Trainingsvorrichtung selbständig, wenn diese in der Aufnahme des Prothesenteils angeordnet ist, wodurch die Bedienung der Trainingsvorrichtung für den Anwender erleichtert wird.

Insbesondere ist die zumindest eine Eingabeeinheit eine RFID - Einheit, wodurch das Anordnen der Trainingsvorrichtung in der Prothese automatisch erkannt wird.

Bevorzugterweise weist der erste Prothesenteil zumindest eine Prothesenmontageeinheit auf, womit die Trainingsvorrichtung einfach an den ersten Prothesenteil angeordnet werden kann.

Bevorzugt ist die zumindest eine Prothesenmontageeinheit komplementär zur ersten Montageeinheit der Trainingsvorrichtung ausgebildet. Damit wird eine Fehlanordnung der Trainingsvorrichtung an den ersten Prothesenteil verhindert und somit die Einsatzdauer der Prothese mit der Trainingsvorrichtung verlängert. Typischerweise werden als Prothesenmontageeinheit und als Montageinheit der Trainingsvorrichtung ein Schienensystem, ein Bajonettverschlusssystem, ein Zapfen-Loch-System oder ein anderes bekanntes Schnellverschlusssystem verwendet, wodurch die Anordnung des Trainingssystems an der Prothese für den Anwender erleichtert wird.

Insbesondere ist die zumindest eine Prothesenmontageeinheit an der Aussenfläche des ersten Prothesenteils angeordnet, wodurch die Anordnung der Trainingsvorrichtung an die Prothese auch im am Anwender angelegten Zustand erfolgen kann.

Bevorzugt ist eine Fixiereinheit zum Fixieren der Trainingsvorrichtung im ersten Prothesenteil vorhanden. Eine ausreichende Fixierung der separierbaren Trainingsvorrichtung am ersten Prothesenteil ist notwendig, um unterschiedliche Bewegungen, wie beispielsweise eine Laufbewegung, eine Bergsteigbewegung, eine Hüpfbewegung u.a. ausführen zu können, ohne dass der Kontakt zwischen den mehreren Stimulatoren der Trainingsvorrichtung mit den Nervenzellenenden des physionomietypischen Nervenarealabschnitts verloren gehen kann.

Vorzugsweise sind externe Sensoren zur Detektion von auf die Prothese einwirkenden Drücke vorhanden, die mit der Trainingsvorrichtung zum Empfangen von Sensordaten verbunden sind, wobei die externen Sensoren in einem weiteren Prothesenteil der Prothese angeordnet sind. Wie hier bereits beschriebenen, weist die Trainingsvorrichtung eine Datenübertragungseinrichtung auf, welche u.a. eine Empfangseinheit zum Empfangen von Sensordaten aufweist. Damit werden die auf die Prothese einwirkenden Drücke mithilfe der in der Trainingsvorrichtung systematisch angeordneten mehreren Stimulatoren auf die Nervenzellenenden im physionomietypischen Nervenarealabschnitt übertragbar, sodass der Anwender die Einwirkung des Drucks auf die Prothese spürt bzw. fühlt.

Insbesondere sind die externen Sensoren an einer Kunstfusssohle angeordnet. Typischerweise sind die externen Sensoren entlang der Ganglinie an der Kunstfusssohle angeordnet, sodass der Anwender der Prothese gefühlsecht Bewegungen mit der Prothese ausführen kann.

Alternativ oder ergänzend sind externe Sensoren zur Detektion von auf die Prothese einwirkenden Kräfte vorhanden, wodurch vom Anwender beispielsweise nicht nur Drücke während einer Gehbewegung wahrgenommen werden können, sondern der Anwedner auch Kräfte auf die Prothese fühlt, welche beispielsweise von einem Stoss auf den seitlichen Prothesenleistenabschnitt erfolgen, bzw. eine Kombination von Drücken und Kräften wahrnehmen kann, wodurch ein realitätsnahes Fühlen mit einer Prothese ermöglicht wird.

Ein weiterer Aspekt der Erfindung betrifft ein Verfahren zum Herstellen einer Prothese, umfassend folgenden Schritt:
- Herstellen einer Aufnahme für das Befestigen einer Trainingsvorrichtung an einem ersten Prothesenteil der Prothese.

Damit lässt sich eine Prothese herstellen, welche individuell auf die Bedürfnisse des Anwenders anfertigen lässt und an die eine Trainingsvorrichtung als modulare Stimulationsvorrichtung angeordnet werden kann. Damit kann diese mit einer Trainingsvorrichtung, welche vorab bereits vom Anwender zu Rehabilitationszwecke verwendet wurde, kombinieren. Damit können die Nervenzellenenden, wie beispielsweise reinnervierte Nervenzellenenden, vom Anwender frühzeitig trainiert werden und dieselbe Trainingsvorrichtung später als modulare Stimulationsvorrichtung in einer Prothese verwendet werden.

Bevorzugt ist die Aufnahme fensterartig ausgebildet, sodass der Anwender die Trainingsvorrichtung einfach in die Aufnahme einsetzen kann und gleichzeitig von aussen seinen eigenen bzw. den physionomietypischen Nervenarealabschnitt sehen kann und diesen gegebenenfalls vorab z.B. einfach reinigen kann.

Insbesondere ist die Trainingsvorrichtung die hier vorliegend beschriebene Trainingsvorrichtung und als eine modulare Stimulationsvorrichtung ausgebildet. Dafür weist die Trainingsvorrichtung eine systematische Anordnung der mehreren Stimulatoren auf, welche einfach vom Anwender mit jeweils zugeordneten Nervenzellenenden verbunden werden können.

Insbesondere wir die hier vorliegend beschriebene Prothese mit dem Verfahren hergestellt.

Bevorzugt wird nach dem Herstellen der Aufnahme, die Trainingsvorrichtung in die Aufnahme eingesetzt und in der Aufnahme fixiert.

Ein weiterer Aspekt der Erfindung betrifft eine Verwendung der hier beschriebenen Trainingsvorrichtung zum Stimulieren von Nervenzellenenden, insbesondere von reinnervierten Nervenzellenenden, in einer hier beschriebenen Prothese. Damit werden die bekanntlich hohen Kosten für einen Prothesenträger minimiert.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Es zeigen dabei:
- Fig. 1: eine erfindungsgemässe Trainingsvorrichtung in einer Schnittansicht,
- Fig. 2: die Trainingsvorrichtung gemäss Fig. 1 in einer Aufsicht,
- Fig. 3: einen der mehreren Stimulatoren der erfindungsgemässen Trainingsvorrichtung in einer detaillierten Darstellung,
- Fig. 4: eine erfindungsgemässe Prothese ohne der Trainingsvorrichtung in einer Schnittansicht,
- Fig. 5: die Kunstfusssohle der Prothese gemäss Fig 4 in einer Aufsicht, und
- Fig. 6: die erfindungsgemässe Prothese gemäss Fig. 4 mit der Trainingsvorrichtung gemäss Fig. 1 in einer perspektivischen Ansicht.

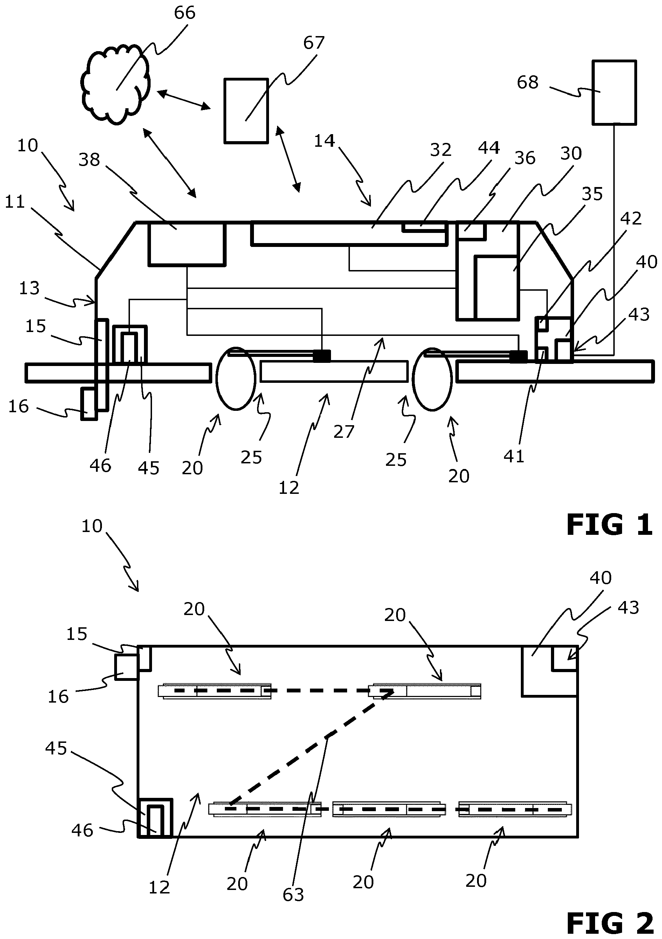

Figur 1 und Figur 2 zeigen die erfindungsgemässe Trainingsvorrichtung 10 zum trainierenden Stimulieren von Nervenzellenenden. Die Trainingsvorrichtung 10 umfasst ein Gehäuse 11 mit einem ersten Gehäuseabschnitt 12, an dem mehrere Stimulatoren 20 zum Stimulieren von physionomietypischen Nervenarealabschnitten angeordnet sind. Dafür weist der erste Gehäuseabschnitt 12 mehrere Gehäuseöffnungen 25 auf, in denen die Stimulatoren 20 abschnittsweise angeordnet sind. Die Trainingsvorrichtung 10 weist an einem zweiten Gehäuseabschnitt 13 des Gehäuses 11 eine Montageeinheit 15 zum Montieren des Gehäuses 11 an einem ersten Prothesenteil einer Prothese sowie eine Positioniereinheit 16 zum Positionieren der Trainingsvorrichtung 10 an einem ersten Prothesenteil einer Prothese. Im Gehäuse 11 ist eine Steuereinrichtung 30 zum Steuern der mehreren Stimulatoren 20 angeordnet. Die Steuereinrichtung 30 umfasst eine Recheneinheit 35, einen Prozessor, sowie eine Speichereinheit 36, welche miteinander und mit den mehreren Stimulatoren 20 elektrisch verbunden sind. In der Recheneinheit 35 werden unterschiedliche Stimulationsprogramme zum Erstellen von Stimulationsbefehlen für einen Stimulationszustand der mehreren Stimulatoren 20 ausgeführt. Weiters werden in der Recheneinheit 35 unterschiedliche Trainingsprogramme zum Erstellen von Trainingsbefehlen für eine Trainingsvorgang für die mehreren Stimulatoren 20 ausgeführt. Die Recheneinheit 20 weist dafür mehrere Rechenalgorithmen auf, mit welchen die Stimulationsbefehle und/oder die Trainingsbefehle erzeugt werden. In der Speichereinheit 36 sind die mehrere Rechenalgorithmen abgespeichert, welche von der Recheneinheit 35 bzw. vom Prozessor nach Bedarf abgerufen werden. Die Steuereinrichtung 30 ist so ausgebildet, dass wahlweise wenigstens ein Trainingsprogramm für Trainingszwecke oder ein Stimulationsprogramm zur Stimulation mittels den mehreren Stimulatoren 20 einstellbar ist.

Die Trainingsvorrichtung 10 weist gemäss dieser speziellen Ausgestaltung eine Datenübertragungseinrichtung 40 mit einer Sendeeinheit 41 und einer Empfangseinheit 42 zum drahtlosen Datenaustausch auf. Die Datenübertragungseinrichtung 40 ist mit der Steuereinrichtung 30 und der Recheneinheit 35 elektrisch verbunden. Die Datenübertragungseinrichtung 40 weist weiter eine Schnittstelle 43 auf, mit der eine Remoteeinrichtung 68, wie beispielsweise ein Computer elektrisch verbunden werden kann und an der ein Trainingsprogramm oder ein Stimulationsprogramm definiert werden kann. Die Sendeeinheit 41 bzw. die Empfangseinheit 42 ist mit einer Cloud 66 oder mit einem mobilen Endgerät 68 (Smartphone, Tablet, usw.) zum Austauschen von Daten, beispielsweise historischen Daten, verbindbar. Die Sendeeinheit 41 ist weiter auch mit einem externen Sensor, beispielsweise einem Sensor an einem Prothesenteil oder einem externen Sensor, wie beispielsweise einem Temperatursensor, einem Luftfeuchtesensor oder einem GPS Sensor usw., zum Austauschen von Daten verbindbar. Die Daten sind an die Recheneinheit 35 übertragbar und werden dort im Rechenalgorithmus verarbeitet, sodass die Steuereinrichtung 30 Stimulationsbefehle und/oder Trainingsbefehle an die mehreren Stimulatoren 20 übertragen kann.

Das Gehäuse 11 weist einen weiteren Gehäuseabschnitt 14 mit der Visualisierungseinrichtung 32 auf, an der einzelne Trainingsdaten bzw. Trainingsbefehle sowie Stimulationsdaten bzw. Stimulationsbefehle und Informationen, wie beispielsweise Nachrichten, gegebenenfalls zum Teil animiert, dargestellt werden. Die Visualisierungseinrichtung 32 ist mit der Steuereinrichtung 30 elektrisch verbunden und erhält von der Steuereinrichtung 30 die Trainingsdaten bzw. Trainingsbefehle sowie Stimulationsdaten bzw. Stimulationsbefehle und Informationen. Die Visualisierungseinrichtung 32 umfasst eine Eingabeeinrichtung 44, die bevorzugt als Touchscreen ausgebildet ist. Die Eingabeeinrichtung 44 umfasst neben einem Hauptschalter (Ein- und Ausschalter) auch einen Auswahlschalter zum Auswählen von vorgegebenen Trainingsprogrammen und/oder Stimulationsprogrammen.

An dem weiteren Gehäuseabschnitt ist eine Energiespeichereinheit 38, eine Batterie bzw. ein Akkumulator angeordnet, welche mit der Steuereinrichtung 30, mit den mehreren Stimulatoren 20 und mit der Erkennungseinrichtung 45 elektrisch verbunden ist. Die Energiespeichereinheit 38 versorgt auch die Visualisierungseinrichtung 32 mit Energie.

Am ersten Gehäuseabschnitt 12 ist eine Erkennungseinrichtung 45, als RFID-Einheit 46, vorgesehen, sodass die Steuereinrichtung 30 automatisch den Bedarf für einen Rehabilitationsmodus (reha mode) und einen Prothesenmodus (aktive mode) erkennt - je nach dem, was unmittelbar angeschlossen ist.

Die mehrere Stimulatoren 20 sind systematisch am ersten Gehäuseabschnitt 12 angeordnet, sodass die mehrere Stimulatoren 20 im Trainingszustand auf deren jeweils zugeordneten Nervenzellenenden einwirken können. Dafür bildet die systematische Anordnung der mehreren Stimulatoren 20 ein Abbild der Ganglinie 63 einer menschlichen Fusssohle ab. Die mehreren Stimulatoren 20 sind blitzförmig am ersten Gehäuseabschnitt 12 angeordnet. Im dargestellten Beispiel in Figur 2 werden dafür fünf Stimulatoren 20 benötigt, um das Abbild der Ganglinie 63 des menschlichen Fusses darzustellen. Die systematische Anordnung der mehreren Stimulatoren 20 erfolgt dabei so, dass jeder Stimulator 20 jeweils zumindest an ein ihm zugeordnetes Nervenzellenende im physionomietypischen Nervenarealabschnitt deckungsgleich übereinstimmt.

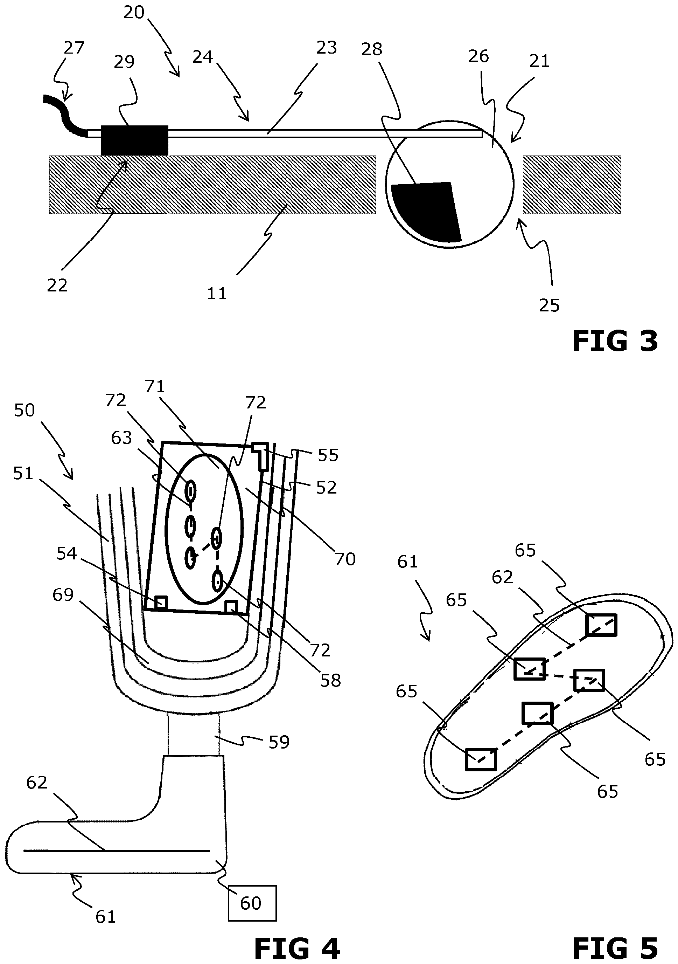

Figur 3 zeigt einen der mehreren Stimulatoren 20 zum Stimulieren von Nervenzellenenden, welche an dem ersten Gehäuseabschnitt 12 angeordnet sind. Der Stimulator 20 weist einen Vibrationserzeuger 21 mit einem Vibrationserzeugergehäuse 26 auf, das mit einem Federelement 23 als Entkopplungselemente 24 verbunden ist. Das Federelement 23 ist ein stabförmiger Federdraht, der an einer Gehäuseseite des Vibrationserzeugergehäuses 26 angeordnet ist. Dafür ist das Federelement 23 mit einem Ende am Vibrationserzeugergehäuse 26 befestigt. Das Federelement 23 entkoppelt die Vibrationen bzw. die Schwingungen des Vibrationserzeugers 21 von der Umgebung. Das andere Ende des Federelements 23 ist mit einem Befestigungsmittel 29 an einem Befestigungsabschnitt 22 des ersten Gehäuseabschnitts 12 befestigt und wird mit den Versorgungsleitungen 27 mit Energie versorgt. Alternativ ist ein weiteres stabförmiges Federelement zwischen dem Befestigungsabschnitt 22 und dem Vibrationserzeugergehäuse 26 angeordnet (nicht gezeigt). Das Vibrationserzeugergehäuse 26 ist zumindest abschnittsweis in die Gehäuseöffnung 25 eingeführt, sodass das Vibrationserzeugergehäuse 26 die Gehäuseöffnung 25 zumindest teilweise durchdringt. Im Vibrationserzeugergehäuse 26 ist ein Exzenterelement 28 angeordnet. Das Exzenterelement 28 ist drehbar bzw. rotierbar im Vibrationserzeugergehäuse 26 gelagert. Das Vibrieren des Vibrationserzeugers 21 wird mit dem Exzenterelement 28 über die wechselnde Richtung der Zentripetalkraft des Exzenterelements 28 verursacht. Der Vibrationserzeugers 21 weist in seinem Vibrationserzeugergehäuse 26 einen Antriebsmotor zum rotatorischen Antreiben des Exzenterelements 28 auf (nicht gezeigt). Der Antriebsmotor ist mit der Energiespeichereinheit 38 verbunden. Alternativ weist der Vibrationserzeuger 21 anstatt des Exzenterelements ein Schwingungselement auf, welches sich entlang einer geraden Führung bewegt und aufgrund der Massenträgheit des Schwingungselements eine Vibration auslöst (nicht gezeigt).

Die Erfindung betrifft auch ein Verfahren zum Betreiben der hier vorliegend beschriebenen Trainingsvorrichtung 10, insbesondere als modulare Stimulationsvorrichtung, umfassend folgende Schritte:
- Anordnen der Trainingsvorrichtung 10 an einem physionomietypischen Nervenarealabschnitt 71 mit Nervenzellenenden, insbesondere mit reinnervierten Nervenzellenenden 72;
- Darstellen der Nervenzellenenden bzw. der ihnen zugeordneten ursprünglichen Wirkungsstelle an der amputierten Extremität des Patienten an einer Visualisierungseinrichtung 32 der Trainingsvorrichtung 10 oder an einem mobilen Endgerät 67.

Zusätzlich werden die mehreren Stimulatoren 20 an der Visualisierungseinrichtung 32 oder am mobilen Endgerät 67 dargestellt, sodass der Anwender der Trainingseinrichtung 10 das Auslösen einer bestimmten Stimulation mit einem bestimmten Stimulator 20 geistig verknüpfen kann und damit der Trainingseffekt verbessert wird.

Anschliessend erfolgt ein Anpassen von zumindest einem Stimulationsbefehl und/oder von zumindest einem Trainingsbefehl an der Eingabeeinrichtung 44 der Visualisierungseinrichtung 32 oder am mobilen Endgerät 67. Dabei wird eines der mehreren Stimulationsprogramme oder eines der mehreren Trainingsprogramme ausgewählt und anschliessend von der Steuereinrichtung 30 ausgeführt. Die mehreren Stimulatoren 20 werden programmgemäss sequenziell bzw. gleichzeitig angeregt, sodass diese die Nervenzellenenden an dem physionomietypischen Nervenarealabschnitt 71 stimulieren.

Ein weiterer Aspekt der Erfindung betrifft die Verwendung der hier vorliegend beschriebenen Trainingsvorrichtung 10 als modulare Stimulationsvorrichtung zum Stimulieren von Nervenzellenenden, insbesondere von reinnervierten Nervenzellenenden .

Die Figur 4 zeigt eine Prothese 50 mit einem Prothesenschaft 51 als ersten Prothesenteil, welche an einer Extremität 69 angeordnet ist. Der Prothesenschaft 51 weist eine Aufnahme 52 zum Aufnehmen einer Trainingsvorrichtung als modulare Stimulationsvorrichtung auf. Die Trainingsvorrichtung ist separierbar an der Aufnahme 52 des Prothesenschafts 51 anordenbar. Die Aufnahme 52 ist fensterartig ausgebildet und umfasst einen Rahmen, wobei die Trainingsvorrichtung darin eingesetzt wird. Die Aufnahme 52 ist im Bereich eines Hautareals 70 der verbliebenen Extremität 69 angeordnet und ermöglicht einen Zugang von aussen auf das Hautareal 70. An diesem Hautareal 70 befindet sich der physionomietypische Nervenarealabschnitt 71 mit den dort angeordneten reinnervierten Nervenzellenenden 72. Die reinnervierten Nervenzellenenden 72 sind in diesem Beispiel nach einer speziellen Matrix, bzw. blitzförmig im physionomietypischen Nervenarealabschnitt 71 angeordnet. Dabei liegen die reinnervierten Nervenzellenenden 72 entlang des Abbilds einer Ganglinie 63. Der Prothesenschaft 51 weist eine Eingabeeinheit 54, als RFID - Einheit auf, welche mit der RFID- Einheit der Trainingsvorrichtung zusammenwirkt und diese im an der Prothese 50 angeordneten Zustand automatisch erkennt. Der Prothesenschaft 51 weist eine Prothesenmontageeinheit 55 auf, womit die Trainingsvorrichtung am Prothesenschaft 51 angeordnet werden kann. Die Prothese 50 weist eine Prothesenkosmetik 59 und einen Kunstfuss 60 mit einer Kunstfusssohle 61 auf, welche ebenso eine Ganglinie 62 der Kunstfusssohle 61 trägt, bzw. entlang der abrollt, wenn der Kunstfuss geht.

Figur 5 zeigt die Kunstfusssohle 61 der Prothese 50. An der Kunstfusssohle 61 sind fünf externe Sensoren 65, welche als Drucksensoren ausgebildet sind, angeordnet. Die externen Sensoren 65 sind blitzförmig an der Kunstfusssohle 61 angeordnet und liegen entlang der Ganglinie 62 der Kunstfusssohle. In der gezeigten Ausführung ist die Form des Abbildes der Ganglinie 63 am physionomietypischen Nervenarealabschnitt 71 der Extremität 69 (Figur 4) gleich der Form der Ganglinie 62 der Kunstfusssohle 61. Es ist jedoch möglich das Abbild der Ganglinie 63 unabhängig zur Ganglinie 62 der Kunstfusssohle 61 zu gestalten. Entscheidend ist dabei nur, dass den jeweiligen Nervenzellenenden im physionomietypischen Nervenarealabschnitt 71 die jeweiligen Stimulatoren 20 der Trainingsvorrichtung 10 zugeordnet werden.

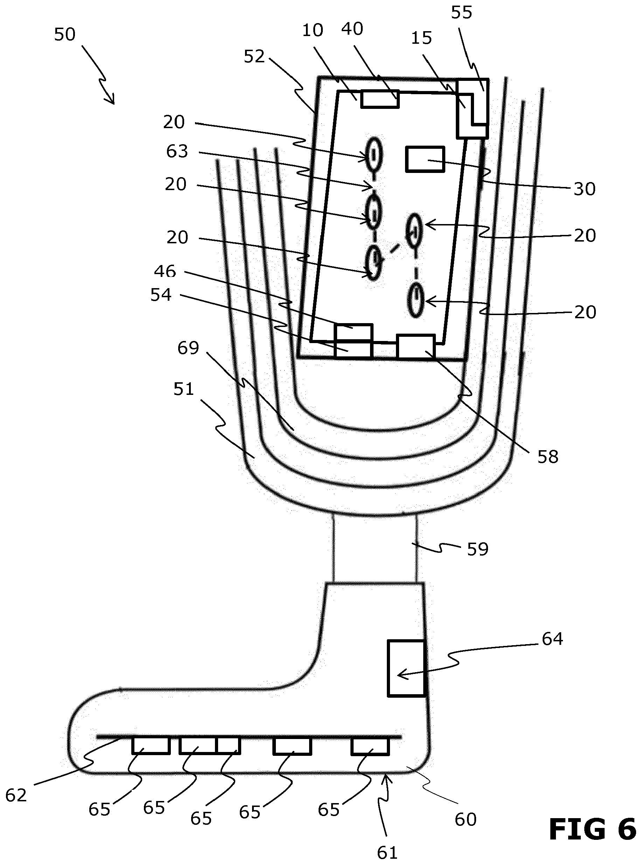

Figur 6 zeigt die hier beschriebene Prothese 50 mit der an der Aufnahme 52 aufgenommenen Trainingsvorrichtung 10. Dabei greift die Montageeinheit 15 der Trainingsvorrichtung 10 komplementär in die Prothesenmontageeinheit 55 am Prothesenschaft 51. Dabei ist die Trainingsvorrichtung 10 mit der Positioniereinheit 16 positionierbar und mit der Fixiereinheit 58 am Prothesenschaft 51 befestigt bzw. fixiert. Die Trainingsvorrichtung 10 ist mit deren systematisch angeordneten Stimulatoren 20 an deren jeweils zugeordneten Nervenzellenenden am physionomietypischen Nervenarealabschnitt 71 der Extremität 69 angeordnet, sodass das Abbild der Ganglinie 63 mit der systematischen Anordnung der mehreren Stimulatoren 20 übereinstimmt.

An der Kunstfusssohle 61 des Kunstfusses 60 sind mehrere externe Sensoren 65 zur Detektion von auf die Prothese 50 einwirkenden Drücke angeordnet. Die mehreren externen Sensoren 65 sind dabei entlang der Ganglinie 62 angeordnet und mit der Sendeeinheit 64 des Kunstfusses 60 elektrisch verbunden. Der Prothese 50 kann auch weitere externe Sensoren zur Detektion von einer auf die Prothese 50 wirkenden Kraft aufweisen. Diese externen Sensoren sind beispielsweise an einem seitlich am Kunstfuss 60 angeordneten Prothesenleistenabschnitt angeordnet und übertragen Sensordaten an die Trainingsvorrichtung 10, welche durch einen Stoss auf diese externen Sensoren im Prothesenleistenabschnitt einwirken (nicht gezeigt). Die Sendeeinheit 64 sendet die Sensordaten von den externen Sensoren 65 und den Sensoren am Prothesenleistenabschnitt an die Empfangseinheit 42 der Datenübertragungseinrichtung 40 der Trainingsvorrichtung 10. Die Sensordaten werden anschliessend an die Steuereinrichtung 30 weitergeleitet.

Wie in Figur 6 dargestellt, weist der Prothesenschaft 51 ein Aufnahme 52 zum Aufnehmen der Trainingsvorrichtung 10 auf. Bei der Fertigung der Prothese 50 wird am Prothesenschaft 51 eine fensterartige Aufnahme 52 zum Befestigen der Trainingsvorrichtung 10 hergestellt. Alternativ wird eine kastenförmige Aufnahme hergestellt, in welcher die Trainingsvorrichtung 10 befestigt wird. Anschliessend wird die Trainingsvorrichtung 10 in die fensterartige Aufnahme 52 eingesetzt wodurch Trainingsvorrichtung 10 mithilfe deren Erkennungseinrichtung 45 von der Eingabeeinheit 54 der Prothese 50 erkannt wird, sodass die Steuereinrichtung 30 automatisch in den Prothesenmodus schaltet. Die Sendeeinheit 64 am Kunstfuss 60 sendet die Sensordaten von den externen Sensoren 65 und den Sensoren am Prothesenleistenabschnitt an die Empfangseinheit 42 der Datenübertragungseinrichtung 40 der Trainingsvorrichtung 10. Die Sensordaten werden anschliessend an die Steuereinrichtung 30 weitergeleitet und dort verarbeitet. In der Recheneinheit 35 der Steuereinrichtung 30 werden die Sensordaten der externen Sensoren in Stimulationsbefehle der jeweiligen zugeordneten Stimulatoren 20 übertragen. Der jeweilige Stimulator 20 stimuliert anschliessend das ihm zugeordneten Nervenzellenende am physionomietypischen Nervenzellenabschnitt 71.

Somit wird die Trainingsvorrichtung 10 in der Regel bei einem von der Prothese 50 separierten Zustand als Trainingsvorrichtung 10 im Rehabilitationsmodus und im an der Prothese 50 angeordneten Zustand im Prothesenmodus verwendet. Abweichungen von dieser Regel können vorgesehen werden, so etwa, wenn der Anwender wünscht, ein Training bei anmontierter Prothese zu erhalten.

### Bezugszeichenliste

- 10: Trainingsvorrichtung
- 11: Gehäuse
- 12: erster Gehäuseabschnitt
- 13: zweiter Gehäuseabschnitt
- 14: weiterer Gehäuseabschnitt
- 15: Montageeinheit
- 16: Positioniereinheit
- 20: Stimulatoren
- 21: Vibrationserzeuger
- 22: Befestigungsabschnitt
- 23: Federelement
- 24: Entkopplungselement
- 25: Gehäuseöffnung
- 26: Vibrationserzeugergehäuse
- 27: Versorgungsleitung
- 28: Exzenterelement
- 29: Befestigungsmittel
- 30: Steuereinrichtung
- 32: Visualisierungseinrichtung
- 35: Recheneinheit
- 36: Speichereinheit
- 38: Energiespeichereinheit
- 40: Datenübertragungseinrichtung
- 41: Sendeeinheit
- 42: Empfangseinheit
- 43: Schnittstelle
- 44: Eingabeeinrichtung
- 45: Erkennungseinrichtung
- 46: RFID Einheit
- 50: Prothese
- 51: Prothesenschaft
- 52: Aufnahme
- 54: Eingabeeinheit
- 55: Prothesenmontageeinheit
- 58: Fixiereinheit
- 59: Prothesenkosmetik
- 60: Kunstfuss
- 61: Kunstfusssohle
- 62: Ganglinie
- 63: Abbild der Ganglinie
- 64: Sendeeinheit
- 65: externe Sensoren
- 66: Cloud
- 67: mobiles Endgerät
- 68: Remoteeinrichtung
- 69: Extremität
- 70: Hautareal
- 71: physionomietypischer Nervenarealabschnitt
- 72: Reinnervierte Nervenzellenende

## Patentansprüche

1. Trainingsvorrichtung (10) zum trainierenden Stimulieren von Nervenzellenenden, insbesondere von reinnervierten Nervenzellenenden (72) eines Fusses, umfassend, ein Gehäuse (11) mit zumindest einem ersten Gehäuseabschnitt (12) sowie mit mehrere Stimulatoren (20) zum Stimulieren von physionomietypischen Nervenarealabschnitten (71), wobei zumindest am ersten Gehäuseabschnitt (12) des Gehäuses (11) mehrere Stimulatoren (20) systematisch angeordnet sind, sodass die mehrere Stimulatoren (20) im Trainingszustand auf die ihnen jeweils zugeordneten Nervenzellenenden einwirken können.

2. Trainingsvorrichtung (10) nach Anspruch 1, **dadurch gekennzeichnet, dass** eine Steuereinrichtung (30) zum Steuern der mehreren Stimulatoren (20) vorhanden ist, welche zumindest ein Trainingsprogramm umfasst und bevorzugt mit einer Visualisierungseinrichtung (32) gekoppelt ist, um dem Patienten eine Trainingsstimulation aus dem zumindest einen Trainingsprogramm optisch darzulegen, und weiter bevorzugt die Steuereinrichtung (30) im Gehäuse (11) angeordnet ist und bevorzugt mit einer externen Remoteeinrichtung (68), insbesondere mit einem mobilen Endgerät (67), steuerbar ist.

3. Trainingsvorrichtung (10) nach Anspruch 1 oder 2, **dadurch gekennzeichnet, dass** die Trainingsvorrichtung (10) eine Datenübertragungseinrichtung (40) zum Übertragen von Daten, aufweist, wobei die Datenübertragungseinrichtung (40) bevorzugt in der Steuereinrichtung (30) angeordnet ist, sodass externe Trainingsdaten und/oder Stimulationsdaten auf die Steuereinrichtung (30) übertragen werden können, und bevorzugt die Datenübertragungseinrichtung (40) eine Schnittstelle (43) umfasst, auf dem ein Anwender Trainingsprogramme und/oder Stimulationsprogramme definieren kann, wobei die Schnittstelle (43) insbesondere eine Eingabeeinrichtung (44) zum Eingeben von Steuerdaten umfasst, wobei die Eingabeeinrichtung (44) bevorzugt an der Visualisierungseinrichtung (32) angeordnet ist, insbesondere als Touchscreen, darin integriert ist.

4. Trainingsvorrichtung (10) nach einem der vorhergehenden Ansprüche, **dadurch gekennzeichnet, dass** die Datenübertragungseinrichtung (40) mit externen Sensordaten (65) einer Prothese (50), bevorzugt einer Kunstfusssohle (61), versorgbar ist und/oder mit mehreren externen Sensoren (65) verbunden ist, und bevorzugt die Datenübertragungseinrichtung (40) mit einer Cloud (66) in einem Netzwerk zum Austauschen von Daten verbunden ist.

5. Trainingsvorrichtung (10) nach einem der vorhergehenden Ansprüche, **dadurch gekennzeichnet, dass** die Steuereinrichtung (30) eine Recheneinheit (35) aufweist, wobei die Recheneinheit (35) zumindest ein Stimulationsprogramm zum Erstellen von Stimulationsbefehlen für einen Stimulationszustand und/oder zumindest ein Trainingsprogramm zum Erstellen von Trainingsbefehlen für eine Trainingsvorgang aufweist, und bevorzugt die Recheneinheit (35) mit der Datenübertragungseinrichtung (40) zum Austausch von Daten, insbesondere von externen Sensordaten, verbunden ist, und insbesondere die Recheneinheit (35) einen Rechenalgorithmus aufweist, welcher im Betriebszustand bevorzugt externe Sensordaten von externen Sensoren (65) einer Kunstfusssohle (61) verarbeitet.

6. Trainingsvorrichtung (10) nach einem der vorhergehenden Ansprüche, **dadurch gekennzeichnet, dass** das Gehäuse (11) zumindest einen zweiten Gehäuseabschnitt (13) aufweist, wobei am zweiten Gehäuseabschnitt (13) eine Montageeinheit (15) zum Montieren des Gehäuses (11) an einem ersten Prothesenteil einer Prothese (50), insbesondere an einem Prothesenschaft (51), vorgesehen ist und bevorzugt eine Positioniereinheit (16) zum Positionieren der Trainingsvorrichtung (10) an dem ersten Prothesenteil einer Prothese (50), und weiter bevorzugt das Gehäuse (11) zumindest einen weiteren Gehäuseabschnitt (14) mit der Visualisierungseinrichtung (32) umfasst und/oder eine Energiespeichereinheit (38) umfasst.

7. Trainingsvorrichtung (10) nach einem der vorhergehenden Ansprüche, **dadurch gekennzeichnet, dass** die Steuereinrichtung (30) so ausgerüstet ist, dass wahlweise ein Trainingsprogramm für Trainingszwecke oder ein Stimulationsprogramm zur Stimulation mittels externer Sensoren (65) einstellbar ist, wobei die Steuereinrichtung (30) insbesondere einen Rehabilitationsmodus und ein Prothesenmodus mittels einer Erkennungseinrichtung (45) automatisch erkennt, wobei bevorzugt die Erkennungseinrichtung (45)eine RFID - Einheit (46) ist.

8. Verfahren zum Betreiben einer Trainingsvorrichtung (10), insbesondere als modulare Stimulationsvorrichtung, nach einem der vorhergehenden Ansprüche, umfassend folgende Schritte:
- Anordnen der Trainingsvorrichtung (10) an einem physionomietypischen Nervenarealabschnitt (71) mit Nervenzellenenden, insbesondere mit reinnervierten Nervenzellenenden (72);
- Darstellen der Nervenzellenenden an einer Visualisierungseinrichtung (32) der Trainingsvorrichtung (10) oder an einem mobilen Endgerät (68), wobei bevorzugt zusätzlich zumindest der Trainingszustand wenigstens eines der mehreren Stimulatoren (20) dargestellt wird.

9. Verwendung der Trainingsvorrichtung (10) nach einem der Ansprüche 1 bis 7, als modulare Stimulationsvorrichtung zum Stimulieren von Nervenzellenenden, insbesondere von reinnervierten Nervenzellenenden (72), bevorzugt mit einem Verfahren nach Anspruch 8.

10. Prothese (50) mit einem ersten Prothesenteil, insbesondere mit einem Prothesenschaft (51) oder einer Prothesenstrumpf, wobei der erste Prothesenteil zumindest eine Aufnahme (52) zum Aufnehmen einer Trainingsvorrichtung als modulare Stimulationsvorrichtung aufweist, insbesondere einer Trainingsvorrichtung (10) nach einem der Ansprüche 1 bis 7, wobei die Trainingsvorrichtung separierbar an der zumindest einen Aufnahme (52) des ersten Prothesenteils angeordnet ist.

11. Prothese (50) nach Anspruch 10, **dadurch gekennzeichnet, dass** der erste Prothesenteil zumindest eine Eingabeeinheit (54) zum Erkennen der Trainingsvorrichtung aufweist, wobei die zumindest eine Eingabeeinheit (54) insbesondere eine RFID - Einheit ist.

12. Prothese (50) nach Anspruch 10 oder 11, **dadurch gekennzeichnet, dass** der erste Prothesenteil zumindest eine Prothesenmontageeinheit (55) aufweist, wobei weiter bevorzugt die zumindest eine Prothesenmontageeinheit (55) komplementär zur Montageeinheit (15) der Trainingsvorrichtung (10) ausgebildet ist sowie insbesondere die zumindest einen Prothesenmontageeinheit (55) an der Aussenfläche des ersten Prothesenteils angeordnet ist, und bevorzugt eine Fixiereinheit (58) zum Fixieren der Trainingsvorrichtung im ersten Prothesenteil vorhanden ist.

13. Prothese (50) nach einem der vorhergehenden Ansprüche, **dadurch gekennzeichnet, dass** externe Sensoren (65) zur Detektion von auf die Prothese (50) einwirkenden Kräften und/oder Drücke vorhanden sind, die mit der Trainingsvorrichtung zum Empfangen von Sensordaten verbunden sind, wobei die externen Sensoren (65) in einem weiteren Prothesenteil der Prothese (50) angeordnet sind, insbesondere an einer Kunstfusssohle (61) angeordnet sind.

14. Verfahren zum Herstellen einer Prothese, insbesondere zum Herstellen einer Prothese (50) nach einem der Ansprüche 10 bis 13, umfassend folgenden Schritt:
- Herstellen einer Aufnahme (52), bevorzugt einer fensterartigen Aufnahme, für das Befestigen einer Trainingsvorrichtung, insbesondere einer Trainingsvorrichtung als eine modulare Stimulationsvorrichtung nach einem der Ansprüche 1 bis 7, an einem ersten Prothesenteil der Prothese (50).

15. Verwendung einer Trainingsvorrichtung (10) zum Stimulieren von Nervenzellenenden, insbesondere von reinnervierten Nervenzellenenden (72), nach einem der Ansprüche 1 bis 7, in einer Prothese (50) nach einem der Ansprüche 10 bis 13.
